# EUROPEAN PATENT SPECIFICATION

(11) **EP 3 087 433 B1**
(45) Date of publication and mention of the grant of the patent: **12.02.2020**
(21) Application number: 14833256.2
(22) Date of filing: 19.12.2014
(51) Int. Cl.: G03G 7/00, G03G 15/14, G03G 15/16

(54) **COSMETIC LASER PRINTER**
KOSMETISCHER LASERDRUCKER
IMPRIMANTE LASER D'ARTICLE COSMÉTIQUE

(30) Priority: 27.12.2013 FR 1363634
(43) Date of publication of application: 02.11.2016
(73) Proprietor: L'Oreal, S.A., 75008 Paris (FR)
(72) Inventor: GIRON, Franck, F-77400 Lagny Sur Marne (FR); SAMAIN, Henri, F-91570 Bievres (FR)
(74) Representative: Nony
(86) International application number: PCT/IB2014/067137
(87) International publication number: WO 2015/097619

(56) References cited:
- EP-A1- 2 090 935
- WO-A1-2010/004526
- US-A1- 2006 093 943
- US-A1- 2011 164 263
- US-B1- 6 190 730

## Description

The present invention relates to making up human keratin materials, especially the skin, lips, nails or coated fibres such as the hair, eyelashes or eyebrows.

The invention relates in particular, but not exclusively, to makeup application by transfer.

### Background

There is an interest in being able to apply personalized makeup. The personalization may relate to the colour of the makeup composition, that the user seeks for example to match exactly to that of the skin, or to a pattern that the user wishes to transfer to the skin.

It has been envisaged to deposit a product directly onto the skin using an inkjet printer head, with the aid of a suitable device.

The invention aims to propose other solutions that make it possible to deposit a cosmetic composition onto human keratin materials, while retaining the possibility of producing, if desired, a complex pattern.

### Summary

According to a first of its aspects, the present invention relates to a process for making up human keratin materials as defined in claim 1 and in the depending claims.

The patent specification discloses a laser printer, which is not part of the claimed invention, for performing the process of the invention for the production of a cosmetic article, characterized in that it is arranged to enable the formation, by electrophotography or magnetophotography, of an image on a support starting from at least one cosmetic toner and in that it is configured to deliver the toner present on the support in a sufficiently free state to enable the removal thereof or transfer thereof by contact with human keratin materials.

Preferably, the printer has no fuser for fusing said toner or has a deactivated or deactivatable fuser.

The term "cosmetic toner" should be understood as meaning a pulverulent cosmetic composition that is compatible with the formation of an image via an electrophotographic or magnetophotographic process as used in laser printers. Preferably, it is a toner that is suitable for electrophotographic use.

The toner is cosmetic in the sense that it is compatible with an application to human keratin materials. Depending on the surface to be made up, the formulation of the toner may be different. For example, for an application to the hair or the nails, it is possible to use certain compounds that might not be used for an application to the lips, for example angular microfacetted pigments capable of injuring the mucous membranes.

This laser printer makes it possible to electrostatically or magnetically deposit a cosmetic composition on a support according to a precise pattern that can be personalized on demand, as a function of an image file sent to the printer.

This laser printer may enable, where appropriate, the user to retouch the pattern once transferred, for example in order to soften the contours thereof, and to smooth the demarcations with the non-madeup area.

This laser printer makes it possible to obtain makeup devices suitable for transfer makeup application by simple contact, without addition of solvent, whether the user seeks to transfer the pattern just after printing or after a longer or shorter period of storage of the device.

This laser printer makes it possible to take advantage, for makeup devices, of the performances of laser printing technology, especially in terms of contrast and precision.

The printer may be a colour or monochrome laser printer.

The cosmetic toner may be contained in a cartridge reservoir. Preferably, this cartridge is removable, in order to allow the toner to be replaced. The cartridge may belong to an assembly comprising a mechanism for metering and homogenizing the toner before the transfer thereof to the drum, and optionally also a reservoir for recovering the excess toner on the drum after formation of the latent image.

Preferably, the printer uses several cosmetic toner cartridges, especially several cartridges of different colours, or one cartridge with multiple compartments enabling the deposition of multiple compositions on the support.

The use of several toners makes it possible, on the one hand, to print a pattern in the colour that is desired, therefore to offer the user the possibility of coating the support with a composition having the colour of their choice, with a view to then removing it or transferring it to the keratin materials. On the other hand, the user may produce complex and precise images, of high resolution, and transfer them to the keratin materials.

The printer may be configured to enable the deactivation, by software means, of the fuser for fusing the toner, when present. This may make the printer compatible for conventional use, with fusing of the toner when it is not a cosmetic toner, in order to print on sheets of paper for example. As a variant, the printer is manufactured without a fuser for fusing the toner. As another variant, the printer has a fuser but the latter is deactivated by physical means.

In particular when the fuser can be deactivated by software means, the printer may be configured in order to recognize the presence of a cosmetic toner cartridge and to deactivate the fuser when the printing takes place from one or more cosmetic toner cartridges.

When the printer has a deactivatable fuser, a mechanism may be incorporated in the printer to prevent contact of the toner layer deposited on the support with one or more rollers capable of rubbing over the composition and affecting the pattern produced. Thus, the toner fuser roller may be introduced in a configuration well away from the support coated with the toner layer.

When the printer has no fuser, the printer may also be arranged in order to prevent any contact of the toner layer deposited on the support with a part of the printer, in order to avoid damaging the printed pattern.

The printer may comprise a fuser that produces a slight fusing of the toner so as to strengthen its cohesion but without the toner actually losing its ability to be removed or transferred. Where appropriate, in this case, the printer may be arranged in order to recognize the presence of a cosmetic toner cartridge and adapt the temperature of the fuser to the nature of the toner so as to preserve the possibility of then removing it or applying it by transfer.

The cosmetic toner cartridge may contain a pulverulent composition comprising coloured particles having a size suitable for use within a toner, and that are compatible with a cosmetic application.

The printer may be arranged in order to transfer the toner deposited on the drum of the printer to a support consisting of a substrate in sheet form and/or borne by a transfer roller. In this case, the transfer roller may be rotated when the latent image is transferred.

### Substrate and transfer surface

In one embodiment example, the substrate used in the invention comprises at least one translucent or transparent area.

The translucent or transparent area allows a user to see through the substrate and thus to visualize more easily the surface to be made up and/or treated before transferring the cosmetic toner. The presence of a translucent or transparent area thus advantageously contributes towards facilitating the production of a precise makeup result on the keratin materials.

The translucent or transparent area of the substrate can be totally or partly superposed with the layer of cosmetic toner, and especially may overlap with it.

The layer of cosmetic toner may be superposed in its entirety on the translucent or transparent area of the substrate. As a variant, only part of the layer of cosmetic toner is superposed on the transparent area of the substrate.

The substrate may be made of a transparent or translucent material. In this case, the translucent or transparent area extends over the entire surface of the substrate.

As a variant, the substrate is opaque over all or part of its surface.

The substrate may comprise a material in sheet form, especially a transparent material.

The substrate may be a flexible sheet or a rigid plate. It may be made of plastic (for example polyethylene or polystyrene).

The substrate is preferentially based on a non-absorbent material, for example a plastic film. The substrate is advantageously non-porous, at least on the face intended to receive the print.

The transfer surface may or may not be flat.

The transfer surface of the substrate may be defined by all or part of: the outer surface of an applicator roller, the surface of an applicator pad, an element in sheet form, a patch, the surface of a porous foam, especially a sponge or a wipe, a coarse brush, a fine brush or a flocked tip.

The applicator roller may have the form of a straight cylinder. In one variant, the roller has the form of an irregular cylinder, for example the form of an hourglass.

In one variant, the roller is "premoulded", i.e. it has an initial non-flat form corresponding to the general form of the area to be made up, for example the negative of the lips, of an eye socket, of an ankle or of a forearm.

In one variant, the substrate is pressed at the time of transfer against an imprint of the area to be made up, so that the transfer surface reproduces the relief of the area to be made up.

The transfer surface is defined, for example, by all or part of the surface of a deformable sheet mounted on the surface of an applicator roller or a pad.

The transfer surface may be elastically deformable. Thus, in a first configuration, the transfer surface may be flat, and, in a second configuration, the transfer surface may be curved.

In one variant, the substrate is configured so that the transfer surface takes a first form, for example substantially flat, during printing, and a second form, different from the first, during the application of the toner to the keratin materials. The second form advantageously corresponds to the form of the surface of the keratin materials intended to be coated with the toner, for example the form of the nails or of a part of the face.

The substrate is preferentially based on a non-absorbent material, for example a plastic film. The substrate is advantageously non-porous, at least on the face intended to receive the print.

In one embodiment example, when the toner is intended to be applied to the cheeks and/or the nails, the substrate may have a thickness of greater than or equal to 1 mm, especially 3 mm, for example ranging from 1 to 5 mm.

In one embodiment example, when the toner is intended to be applied to the area around the eyes and/or to the lips, the substrate may have a thickness of greater than or equal to 3 mm, especially 1 cm, for example ranging from 3 mm to 20 mm.

In one embodiment example, when the toner is intended to be applied to the nose and/or in the area of the ears, the substrate may have a thickness of greater than or equal to 1 cm, especially 3 cm, for example ranging from 1 to 4 cm.

Thus, the substrate advantageously has a thickness adapted to the area of keratin materials to be made up.

The thickness of the substrate corresponds to its maximum dimension measured perpendicular to the transfer surface.

The substrate may have a variable thickness.

The substrate may be premoulded.

In one embodiment example, the substrate comprises a printed instruction. The instruction states, for example, the nature of the keratin materials intended to be made up with the toner or illustrates to scale, enlarged, reduced or otherwise and "right-side up" the pattern deposited "wrong-side up" on the substrate.

In one embodiment example, the transfer surface is detachable from a part of the substrate.

The substrate may be reusable.

For example, printing is performed on the substrate, which is accessible for the transfer, but does not leave the printer. Thus, after use, the printer can reintegrate the substrate, clean it and make it ready for a new print. When the support on which the composition is deposited is a roller, the latter may then be removed from the printer in order to apply the composition to the keratin materials by transfer, by making the roller roll in contact with the skin for example. This roller may be firmly attached to a gripping portion that facilitates the handling thereof.

Another subject of the patent specification is a cartridge of cosmetic toner, which is not part of the claimed invention, characterized in that it comprises a case containing a cosmetic toner, the case being designed to be received inside a printer according to the patent specification.

Another subject of the patent specification is a cosmetic toner., which is not part of the claimed invention. This toner may comprise, besides a colouring agent, a compound for controlling the electrical charge, a particular additional filler, a lubricant, a wax and/or a binder. Preferably, the particles of the toner have a mean size D₅₀ of between 1 and 16 µm.

Another subject of the patent specification is a process, which is not part of the claimed invention, for preparing a support for performing the making up process of the invention bearing a cosmetic composition to be applied to a human keratin material, comprising a step that consists in applying said composition to the support with the aid of a laser printer according to the patent specification.

The making up process of the invention may comprise a step that consists in choosing the pattern to be printed as a function of the region to be treated.

The pattern to be printed may comprise a colour gradation. As a variant, the pattern to be printed is in the form of a flat tint.

The process may comprise a step that consists in selecting a printing colour from a palette displayed on a screen.

The pattern to be printed may reproduce any pattern of use for makeup, for example a pattern that imitates a skin texture, to be applied by transfer.

The printing may also follow geometric rectification rules. In so far as the transfer surface is deformable, during the application, the pattern will be geometrically deformed (for example extension in one of the two dimensions). As a result, the pattern is printed with a geometrical deformation (in the present case reduction along the deformable dimension(s)) such that, after application, the pattern is at the desired scale. Geometric rules, either universal or specific, may be applied to the pattern to be printed on the transfer surface so that the pattern has the desired form after transfer onto the area of the keratin materials to be treated. The use of such rectification rules is particularly advantageous with a substrate that has a transfer surface bearing reliefs, in particular in order to match an imprint, as will be seen later. Use may be made in particular of specific geometric rules adapted to the area to be treated and/or to the desired pattern.

The process for making up human keratin materials comprises the step that consists in preparing a support by using the preparation process according to the patent specification as defined above, then in applying the composition present on the support to said human keratin materials, for example the skin, lips, eyelashes or eyebrows, the hair or nails.

The making up process preferably comprises the application of the composition by transfer, by applying the support coated with composition to said human keratin materials.

The application may also take place, as a variant, by removing the composition on the support with the aid of an applicator, for example a flocked tip, a sponge or a foam.

The making up process may comprise the acquisition of an image of the area to be made up prior to the preparation of the support, and a step that consists in printing the pattern as a function of the image thus acquired. This makes it possible, for example, to adapt the contour of the pattern to the relief and/or to the colour of the area to be made up.

After transfer of the toner to the keratin materials, the latter may be covered with the toner of a protective coating, in particular deposited by spraying. It may be a colourless resin.

Another subject of the patent specification, which is not part of the claimed invention, is a device for packaging and applying a cosmetic composition, comprising a support coated with a cosmetic toner, preferably forming a pattern, in particular a support on which a cosmetic composition has been deposited with a printer according to the patent specification. The pattern present on the support may be polychromatic and correspond for example to a gradation.

Another subject of the patent specification, which is not part of the claimed invention, is a support comprising a layer of cosmetic toner deposited by an electrophotographic or magnetophotographic process according to a predefined pattern.

The invention may be better understood from reading the following detailed description of non-limiting implementation examples thereof and from examining the appended drawing, in which:
- Figure 1 schematically illustrates an electrophotographic printing process that may be carried out within a laser printer,
- Figure 2 represents an example of a support coated with a cosmetic composition in accordance with the invention.

### Laser printer

An example of a laser printer is disclosed in application US 2006/0093943 A1.

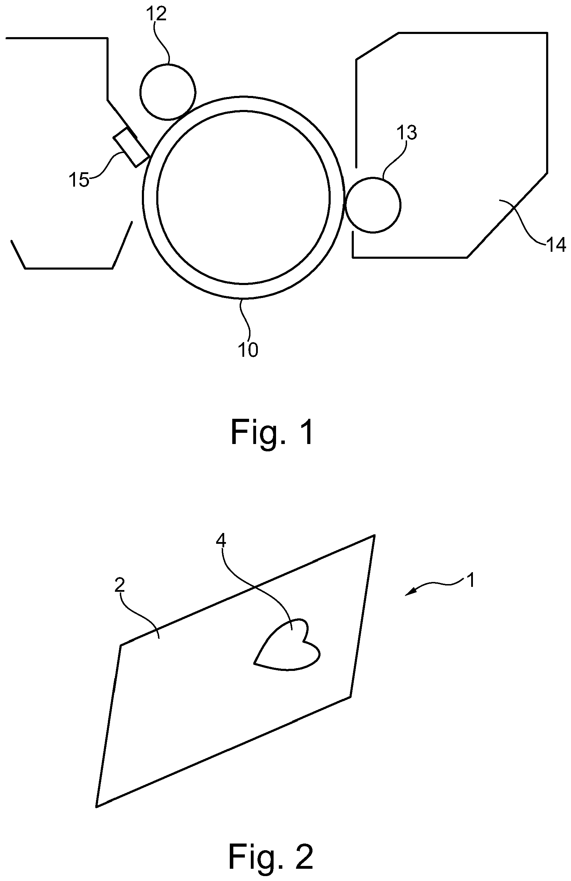

As is known, a laser printer that uses an electrophotographic printing process comprises, as illustrated in Figure 1, a drum 10 coated with a photoreceptor which is used to receive an electrostatic latent image, an element 12 for electrically charging the photoreceptor before the exposure thereof to the laser, an element 13 for developing the electrostatic latent image with a toner removed from a cartridge 14 and a cleaning element 15 for cleaning the drum after transfer of the developed image.

In Figure 1, the following have not been represented: the laser imager that forms the electrostatic latent image on the drum or the mechanism which transfers the developed image onto an intermediate transfer element, for example a strip circulating in closed loop form, before contact of this intermediate element with a support such as a sheet of paper.

The electrostatic image is, as is widely known, created as a function of the areas where the laser has not irradiated the photoreceptor. The toner particles are selectively deposited on the drum, according to the distribution of the electrostatic charges, in order to reproduce the image to be obtained.

In one variant, the image is formed by magnetophotography, the photoreceptor and the toner being magnetic. Publications describe this process, for example application EP 2 090 935 A1 in paragraphs [0009] to [0014].

A conventional laser printer also comprises a fuser comprising a heat roller and a means for controlling the temperature of this heat roller. In standard operation, the fuser module is turned on for each printing operation and fuses a binder of the toner particles to a support, for example a sheet of paper.

Within a printer according to the patent specification, the fuser may be absent or deactivated, either by a physical intervention, or by software means. The fuser is not rendered necessary due to the fact that the toner has not been fused, since it may remain pulverulent so as to be able to be removed and to be able to transfer.

As a variant, the printer retains a fuser but the fusing temperature is chosen so as not to prevent the subsequent removal of the toner deposited on the support, for application to keratin materials.

Preferably, the laser printer used operates electrophotographically, rather than magnetophotographically.

Preferably also, the laser printer is a polychrome printer, rather than a monochrome printer.

### Toner

The toner according to the patent specification is suitable for a cosmetic application. It is thus non-toxic with respect to the region to which it is applied.

The toner may already be used for conventional laser printing, if the formulation of this toner is suitable for use in cosmetics.

The choice of the known toners that can be used may be wider for certain applications, for example to the hair, eyelashes or nails, than for others, for example to the skin or lips.

### Binder

A toner conventionally comprises a binder, which is melted during fusing in order to fix the toner to the support.

The toner according to the patent specification may or may not comprise such a binder, which is not melted or is only very partially melted so as not to prevent the removal or transfer for the makeup application.

Among the binders that can be used, mention may be made, among those conventionally used, of those which are compatible with an application to human keratin materials.

Use may especially be made of the binders disclosed in the publication WO 2007/134171, which describes a toner compatible with a food use, and in particular a polymer chosen from polyvinyl acetate/polyvinylpyrrolidone copolymers (such as Kollidon® SR or Kollidon® VA64 from BASF), poly(N-vinyl-2-pyrrolidone)s, polyethylenes, polypropylenes, polyamides and polyurethanes.

### Colouring agents

The toner according to the patent specification comprises a colouring agent, which may be incorporated into the binder, where appropriate.

The colouring agent may be chosen from the dyes and pigments conventionally used in cosmetics, subject to the compatibility thereof with the printing process used within the printer.

This colouring agent may in particular be chosen from those mentioned in application WO 2007/134171, in particular from natural dyes such as curcumin, indigo, dyes derived from logwood, orcein, authoayanins, caramel, carmine, annatto, beta-carotenes, saffron, chlorophyll, and vegetable black.

### Agent for controlling the charge

The toner may comprise an agent for controlling the charge, intended to promote the electrostatic transfer of the toner to the drum.

This agent may be chosen from those conventionally used, subject to the compatibility thereof with a cosmetic use.

Use may be made of those mentioned in application WO 2007/134171, namely quaternary ammonium salts, benzalkonium chloride, benzethonium chloride, cetrimide (trimethytetradecylammonium bromide), cyclodextrins, silica, aluminium oxide, titanium dioxide, ferrite and carbon black.

### Waxes

The toner may comprise a wax, especially when a slight fusing of the toner is carried out, or in order to improve the hold on the keratin materials.

The term "wax" means a lipophilic compound, which is solid at room temperature (25°C), with a reversible solid/liquid change of state, with a melting point of greater than or equal to 30°C, which may be up to 120°C. By bringing the wax to the liquid state (melting), it is possible to make it miscible with the oils that may be present and to form a microscopically homogeneous mixture, but on returning the temperature of the mixture to room temperature, recrystallization of the wax in the oils of the mixture is obtained. The melting point of the wax may be measured using a differential scanning calorimeter (DSC), for example the calorimeter sold under the name DSC 30 by the company Mettler.

The waxes may be hydrocarbon-based waxes, fluoro waxes and/or silicone waxes, and may be of plant, mineral, animal and/or synthetic origin. In particular, the waxes have a melting point of greater than 25°C and better still greater than 45°C. As wax that may be used in the colouring ink, mention may be made of beeswax, carnauba wax or candelilla wax, paraffin, microcrystalline waxes, ceresin or ozokerite; synthetic waxes such as polyethylene waxes or Fischer-Tropsch waxes, silicone waxes such as alkyl or alkoxy dimethicones containing from 16 to 45 carbon atoms.

### Particulate additive

The toner preferably comprises a particulate additive in order to improve the fluidity, the developability and the sensitivity to electrostatic charges thereof.

The particles of the additive may have a mean size ranging from 5 nm to 2 µm, better still from 5 nm to 500 nm, and their specific surface area preferably ranges from 20 to 500 m²/g according to the BET method.

The proportion of the additional filler preferably ranges from 0.01% to 5% by weight and better still from 0.01% to 2% by weight relative to the total weight of the toner.

The additional filler may be chosen from particles of silica, alumina, titanium dioxide, zinc oxide, clay, mica, diatomaceous earths, red iron oxide, magnesium oxide, zirconium oxide, calcium carbonate, silicon carbide and silicon nitride.

Preferably, use is made of silica and/or titanium dioxide, preferably in combination and preferably silica and/or titanium dioxide that have been rendered hydrophobic. The size of the silica and/or titanium dioxide particles is preferably less than 50 nm.

Preferably, the mixture of hydrophobic silica and hydrophobic titanium dioxide is in a proportion by weight of between 0.3% and 1.5% relative to the total weight of the toner.

### Preparation of the toner

It is desirable for the toner particles to have a mean size D₅₀ between 1 and 16 µm, better still between 3 and 10 µm, with as narrow a distribution as possible about a mean value so as to improve the printing quality.

The toner particles may be prepared by any suitable method, for example involving milling and pulverizing, as disclosed in paragraphs [0075] to [0079] of US 2006/0093943 A1 or in the examples given in application WO 2007/134171.

### Magnetic carrier

The toner according to the patent specification may be used in combination with a magnetic carrier such as ferrite or iron particles, as disclosed in paragraphs [0082] and [0083] of US 2006/0093943 A1.

### Support

The printing support may be of any suitable type and may in particular consist of a sheet material such as a paper or a plastic film.

Represented in Figure 2 is a support 2 in the form of sheet material, on one face of which a pattern 4 has been printed electrophotographically using a laser printer according to the patent specification.

The support may be, where appropriate, deformable and flexible so as to more readily match the relief of the keratin materials onto which the pattern must be transferred.

The support may be impermeable to water, in order to facilitate the transfer of the pattern after wetting of the surface onto which the pattern must be transferred.

### Modes of application

The cosmetic toner(s) deposited by printing may be transferred by bringing them into contact with the area to be made up and pressing thereon.

The transfer may be carried out dry, without prior wetting of the area to be made up or of the printed pattern.

As a variant, a transfer compound is applied to at least one of the pattern and of the area to be made up, for example an adhesive, with the view to facilitating the transfer and improving the hold of the composition transferred to the made up area.

In one embodiment example, the printing is performed directly onto a non-flat transfer surface, corresponding for example to the outer surface of a roller. This roller is for example applied against the intermediate transfer element of the printer, and turns in contact therewith in order to receive the pattern. Next, the roller is moved onto the skin for the transfer.

### Printed pattern

The pattern formed on the surface of the support may be of any type.

The process may comprise a step of choosing and/or making the pattern by a user and of transmitting, by means of a machine connected to the printer that performs the printing, information relating to this pattern.

The machine may be a computer, an advanced portable telephone, also known as a "smartphone", or a tablet computer. The machine may be connected physically and/or by means of a data exchange network to the said printer.

The toner or toners are deposited in the form of dots raster and/or of raster lines, so as to form a halftone image, for example a monochromatic or polychromatic image.

The pattern may reproduce the appearance of relief and/or colour heterogeneities of the skin, for example freckles or skin texture.

The pattern may be coloured when observed under white light in the visible region (400 nm - 800 nm). As a variant, the pattern is colourless under white light in the visible region, but may appear coloured when submitted to a chemical and/or energy stimulus, such as exposure to UV (365 nm - 400 nm).

The printing may use only toners that correspond to primary colours. As a variant, the printing uses both toners of primary colours and at least one toner of non-primary colour.

The printing may be monochromatic, but preferably it is carried out as three-colour printing or as four-colour printing.

The pattern obtained by printing may comprise several areas of different colours, for example according to a gradation. As a variant, the pattern obtained by printing is a flat tint.

### Test 1

Use is made of an HP LaserJet Pro 400 M451 NW printer, modified to remove the heat roller.

The electronic system is modified to prevent an operating error following the removal of the heat roller. The thermistor used for measuring the temperature is especially replaced with a resistor simulating a heat roller temperature that is normally encountered.

Removal of the heat roller makes it possible to minimize the mechanical stress during printing without melting the toners.

The following preparation is used as cosmetic toners:
A toner of an HP Laser jet pro Color M451nw printer is taken. After opening, the existing powder is removed and replaced with a powder (40 g) containing 6 g of ferrite, 33 g of carbon black powder and 1 g of calcium carbonate, rendered pulverulent by blending with aeration.

Printing is performed on a sheet of transparent type for a laser printer.

The printed pattern is applied by transfer onto the skin just after printing. The sheet is placed on the skin with a pressure of 50 g per cm² for 5 seconds. The sheet is then removed.

To complete the treatment, a composition containing a resin is sprayed on, at a distance of 30 cm. To do this, an Elnett brand hair lacquer is used. It is left at rest for 1 minute. At the end of which the treatment is complete.

### Test 2

Use is made of an OKI C711WT printer, modified to remove the heat roller.

The electronic system is modified to prevent an operating error following the removal of the heat roller. The thermistor used for measuring the temperature is especially replaced with a resistor simulating a heat roller temperature that is normally encountered.

Removal of the heat roller makes it possible to minimize the mechanical stress during printing without melting the toners.

Use is made, as cosmetic toners, of the standard toners of the OKI C711WT printer (Cyan P/N 44318607; Magenta P/N 44318606; Yellow P/N 44318605; White P/N 44318657).

Printing is performed on a sheet of transparent type for a laser printer.

The printed pattern is applied by transfer onto the skin just after printing. The sheet is placed on the skin with a pressure of 50 g per cm² for 5 seconds. The sheet is then removed.

To complete the treatment, a composition containing a resin is sprayed on, at a distance of 30 cm. To do this, an Elnett brand hair lacquer is used. It is left at rest for 1 minute. At the end of which the treatment is complete.

## Claims

1. Process for making up human keratin materials, comprising the steps that consist in:
- preparing a support (2) bearing a cosmetic composition to be applied to a human keratin material by applying said composition to the support (2) with the aid of a laser printer that comprises a cartridge of cosmetic toner, comprising a pulverulent cosmetic composition, and the laser printer being arranged to enable the formation, by electrophotography or magnetophotography, of an image on a support (2) starting from at least one cosmetic toner and that is configured to deliver the toner present on the support (2) in a pulverulent state which is a sufficiently free state to enable the removal thereof or transfer thereof by contact with human keratin materials,
the laser printer being arranged in order to transfer the toner deposited on a drum of the printer to a substrate in sheet form or in roller form,
- applying the composition present on the support to said human keratin materials, the application of the composition taking place by transfer, by applying the support coated with composition to said human keratin materials and/or the application taking place by removing the composition on the support using an applicator.

2. Process according to Claim 1, the printer having no fuser for fusing said toner or having a deactivated or deactivatable fuser.

3. Process according to Claim 1 or 2, the printer being configured in order to enable the deactivation, by software means, of the fuser for fusing the toner.

4. Process according to any one of Claims 1 to 3, the printer being configured in order to recognize the presence of a cosmetic toner cartridge and to deactivate the fuser when the printing takes place from one or more cosmetic toner cartridges and/or to adapt the temperature of the fuser to the nature of the toner.

5. Process according to any one of Claims 1 to 4, the printer comprising several cartridges of cosmetic toner, especially several cartridges of different colours.

6. Process according to any one of Claims 1 to 5, the cosmetic toner comprising a colouring agent, a compound for controlling the electrical charge and a particular additional filler.

7. Process according to any one of the preceding claims, comprising a step that consists in choosing a pattern (4) to be printed as a function of the region to be treated.

8. Process according to the preceding claim, the pattern (4) to be printed comprising a colour gradation.

9. Process according to Claim 7, the pattern (4) to be printed being in the form of a flat tint.

10. Process according to any one of the preceding claims, comprising a step that consists in selecting a printing colour from a palette displayed on a screen.

11. Process according to Claim 7 to 9, the pattern (4) to be printed reproducing a skin texture to be applied by transfer.

12. Process according to any one of the preceding claims, the application of the composition taking place by transfer, by applying the support coated with composition to said human keratin materials.

13. Process according to Claims 1 to 11, the application taking place by removing the composition on the support using an applicator.

14. Process according to any one of the preceding claims, comprising the acquisition of an image of the area to be made up prior to the preparation of the support, and a step that consists in printing a pattern as a function of the image thus acquired.

## Patentansprüche

1. Verfahren zum Schminken von menschlichen Keratinmaterialien, das die folgenden Schritte umfasst:
- Anfertigen eines Trägers (2), der eine kosmetische Mischung trägt, die auf ein menschliches Keratinmaterial aufgebracht werden soll, indem die Mischung auf den Träger (2) mit Hilfe eines Laserdruckers aufgebracht wird, der eine Patrone aus kosmetischem Toner umfasst, der eine pulverförmige kosmetische Mischung enthält, wobei der Laserdrucker so ausgelegt ist, dass er durch Elektrophotographie oder Magnetophotographie die Ausbildung eines Bildes auf einem Träger (2) ermöglicht, wobei mit wenigstens einem kosmetischen Toner begonnen wird, und konfiguriert ist, den Toner zuzuführen, der auf dem Träger (2) in einem pulverförmigen Zustand vorliegt, der ein ausreichend freier Zustand ist, um das Entfernen oder Übertragen durch Kontakt mit menschlichen Keratinmaterialien zu ermöglichen,
wobei der Laserdrucker so ausgelegt ist, dass er den Toner, der auf einer Trommel des Druckers abgeschieden wurde, auf ein Substrat in Folienform oder in Walzenform überträgt,
- Aufbringen der Mischung, die auf dem Träger vorliegt, auf menschliche Keratinmaterialien, wobei das Aufbringen der Mischung durch Übertragen stattfindet, indem der Träger, der mit der Mischung beschichtet ist, auf die menschlichen Keratinmaterialien aufgebracht wird und/oder indem das Aufbringen durch Entfernen der Mischung auf dem Träger unter Verwendung eines Applikators stattfindet.

2. Verfahren nach Anspruch 1, wobei der Drucker kein Fixierelement zum Fixieren des Toners aufweist oder ein deaktiviertes oder deaktivierbares Fixierelement aufweist.

3. Verfahren nach Anspruch 1 oder 2, wobei der Drucker so konfiguriert ist, dass er mittels Software die Deaktivierung des Fixierelements zum Fixieren des Toners ermöglicht.

4. Verfahren nach einem der Ansprüche 1 bis 3, wobei der Drucker so konfiguriert ist, dass er das Vorhandensein einer Patrone mit kosmetischem Toner erkennt und dass er das Fixierelement deaktiviert, wenn das Drucken aus einer oder mehreren Patronen mit kosmetischem Toner erfolgt und/oder die Temperatur des Fixierelements an die Art des Toners anpasst.

5. Verfahren nach einem der Ansprüche 1 bis 4, wobei der Drucker mehrere Patronen mit kosmetischem Toner, insbesondere mehrere Patronen mit unterschiedlichen Farben umfasst.

6. Verfahren nach einem der Ansprüche 1 bis 5, wobei der kosmetische Toner ein Färbemittel, eine Mischung zum Steuern der elektrischen Ladung und einen zusätzlichen Partikelfüllstoff umfasst.

7. Verfahren nach einem der vorhergehenden Ansprüche, das einen Schritt umfasst, der im Wählen eines Musters (4), das gedruckt werden soll, in Abhängigkeit von dem zu behandelnden Bereich besteht.

8. Verfahren nach dem vorhergehenden Anspruch, wobei das zu druckende Muster (4) eine Farbabstufung umfasst.

9. Verfahren nach Anspruch 7, wobei das zu druckende Muster (4) die Form einer matten Tinte hat.

10. Verfahren nach einem der vorhergehenden Ansprüche, das einen Schritt umfasst, der im Auswählen einer Druckfarbe aus einer Palette besteht, die auf einem Bildschirm angezeigt wird.

11. Verfahren nach Anspruch 7 bis 9, wobei das zu druckende Muster (4) eine Hauttextur wiedergibt, die durch Übertragen aufgebracht werden soll.

12. Verfahren nach einem der vorhergehenden Ansprüche, wobei das Aufbringen der Mischung durch Übertragen stattfindet, indem der Träger, der mit der Mischung beschichtet ist, auf die menschlichen Keratinmaterialien aufgebracht wird.

13. Verfahren nach Anspruch 1 bis 11, wobei das Aufbringen stattfindet, indem die Mischung auf dem Träger unter Verwendung eines Applikators entfernt wird.

14. Verfahren nach einem der vorhergehenden Ansprüche, das die Erfassung eines Bildes des Bereichs, der geschminkt werden soll, vor der Anfertigung des Trägers und einen Schritt, der im Drucken eines Musters in Abhängigkeit von dem so erfassten Bild besteht, umfasst.

## Revendications

1. Procédé de maquillage de matières kératiniques humaines, comportant les étapes consistant à :
- préparer un support (2) portant une composition cosmétique à appliquer sur une matière kératinique humaine en appliquant ladite composition sur le support (2) à l'aide d'une imprimante laser qui comporte une cartouche de toner cosmétique, comportant une composition cosmétique en poudre, et l'imprimante laser étant agencée pour permettre la formation, par électrophotographie ou magnétophotographie, d'une image sur un support (2) à partir d'au moins un toner cosmétique et qui est configurée pour délivrer le toner présent sur le support (2) dans un état pulvérulent qui est un état suffisamment libre pour permettre son prélèvement ou son transfert par contact avec des matières kératiniques humaines,
l'imprimante laser étant agencée pour transférer le toner déposé sur un tambour de l'imprimante sur un substrat en feuille ou en rouleau,
- appliquer la composition présente sur le support sur lesdites matières kératiniques humaines, l'application de la composition s'effectuant par transfert, en appliquant le support revêtu de composition sur lesdites matières kératiniques humaines et/ou l'application s'effectuant en prélevant la composition sur le support à l'aide d'un applicateur.

2. Procédé selon la revendication 1, l'imprimante étant sans four de cuisson du toner ou à un four désactivé ou désactivable.

3. Procédé selon la revendication 1 ou 2, l'imprimante étant configurée pour permettre la désactivation, par voie logicielle, du four de cuisson du toner.

4. Procédé selon l'une quelconque des revendications 1 à 3, l'imprimante étant configurée pour reconnaître la présence d'une cartouche de toner cosmétique et désactiver le four lorsque l'impression s'effectue à partir d'une ou de plusieurs cartouches de toner cosmétique et/ou adapter la température du four à la nature du toner.

5. Procédé selon l'une quelconque des revendications 1 à 4, l'imprimante comportant plusieurs cartouches de toner cosmétique, notamment plusieurs cartouches de couleurs différentes.

6. Procédé selon l'une quelconque des revendications 1 à 5, le toner cosmétique comportant un agent de coloration, un composé de contrôle de la charge électrique et une charge additionnelle particulaire.

7. Procédé selon l'une quelconque des revendications précédentes, comportant une étape consistant à choisir un motif (4) à imprimer en fonction de la région à traiter.

8. Procédé selon la revendication précédente, le motif (4) à imprimer comportant un dégradé de couleurs.

9. Procédé selon la revendication 7, le motif (4) à imprimer étant sous la forme d'un aplat de couleur.

10. Procédé selon l'une quelconque des revendications précédentes, comportant une étape consistant à sélectionner une couleur d'impression sur une palette affichée sur un écran.

11. Procédé selon les revendications 7 à 9, le motif (4) à imprimer reproduisant un grain de peau à appliquer par transfert.

12. Procédé selon l'une quelconque des revendications précédentes, l'application de la composition s'effectuant par transfert, en appliquant le support revêtu de composition sur lesdites matières kératiniques humaines.

13. Procédé selon les revendications 1 à 11, l'application s'effectuant en prélevant la composition sur le support à l'aide d'un applicateur.

14. Procédé selon l'une quelconque des revendications précédentes, comportant l'acquisition d'une image de la zone à maquiller préalablement à la préparation du support, et une étape consistant à imprimer un motif en fonction de l'image ainsi acquise.
